(19) Europäisches Patentamt

European Patent Office

Office européen des brevets

(11) Veröffentlichungsnummer: **0 266 501 B1**

(12) **EUROPÄISCHE PATENTSCHRIFT**

(45) Veröffentlichungstag der Patentschrift: **15.04.92**

(21) Anmeldenummer: **87112026.7**

(22) Anmeldetag: **19.08.87**

(51) Int. Cl.⁵: **G01N 27/12**, G01N 27/417, G01N 33/00, F02D 41/02, F02D 41/30

(54) Verfahren und Schaltungsanordnung zur Erkennung der Betriebsbereitschaft einer Sauerstoffmesssonde.

(30) Priorität: **30.10.86 DE 3637029**
**24.12.86 DE 3644472**

(43) Veröffentlichungstag der Anmeldung:
**11.05.88 Patentblatt 88/19**

(45) Bekanntmachung des Hinweises auf die Patenterteilung:
**15.04.92 Patentblatt 92/16**

(84) Benannte Vertragsstaaten:
**DE FR GB IT SE**

(56) Entgegenhaltungen:
EP-A- 0 009 305      EP-A- 0 099 545
DE-A- 3 118 522      DE-A- 3 644 472
US-A- 4 132 193      US-A- 4 208 993

(73) Patentinhaber: **VDO Adolf Schindling AG**
**Gräfstrasse 103**
**W-6000 Frankfurt/Main 90(DE)**

(72) Erfinder: **Reisch, Wolfgang**
**Römerberg 5**
**W-6465 Biebergemünd-Bieber(DE)**
Erfinder: **Lukas, Bernard**
**Hermann-Löns-Strasse 16**
**W-6096 Raunheim(DE)**
Erfinder: **Blümel, Thomas**
**Zum Feldberg 49**
**W-6384 Schmitten 1(DE)**
Erfinder: **Mausner, Eberhard**
**Rotherweingartenweg 42a**
**W-6232 Bad Soden/Ts(DE)**

(74) Vertreter: **Klein, Thomas, Dipl.-Ing. (FH)**
**Sodener Strasse 9 Postfach 6140**
**W-6231 Schwalbach a. Ts.(DE)**

**Beschreibung**

Die Erfindung betrifft ein Verfahren und eine Schaltungsanordnung zur Erkennung der Betriebsbereitschaft einer Sauerstoffmeßsonde, die im Abgaskanal einer Brennkraftmaschine angeordnet ist und zusammen mit einer Regeleinrichtung zur Regelung der Gemischaufbereitung für die Brennkraftmaschine dient. Zur Erzielung möglichst schadstofffreier Abgase sind Regeleinrichtungen für Brennkraftmaschinen bekannt, bei denen der Sauerstoffgehalt im Abgaskanal gemessen und ausgewertet wird. Hierzu sind Sauerstoffmeßsonden, sogenannte Lambda-Sonden, bekannt, die nach dem Prinzip der Ionenleitung durch einen Festelektrolyten infolge einer Sauerstoffpartialdruckdifferenz arbeiten und entsprechend dem im Abgas vorliegenden Sauerstoffpartialdruck ein Spannungssignal abgeben, das beim Übergang vom Sauerstoffmangel zum Sauerstoffüberschuß einen Spannungssprung aufweist.

Der Innenwiderstand der bekannten Sauerstoffmeßsonden ist jedoch bei geringen Temperaturen derart groß, daß das von der Sauerstoffmeßsonde abgegebene Signal nach einem Kaltstart und während einer Warmlaufphase der Brennkraftmaschine nicht ausgewertet werden kann. Bei bekannten Einrichtungen zur Regelung der Gemischaufbereitung ist daher bis zum Erreichen der Betriebsbereitschaft der Sauerstoffmeßsonde eine vom Ausgangssignal der Sauerstoffmeßsonde unabhängige Steuerung vorgesehen. Erst wenn die Sauerstoffmeßsonde ihre Betriebsbereitschaft erreicht hat, wird deren Ausgangssignal zur Regelung des Brennstoff-Luftverhältnisses herangezogen.

Bei einem bekannten Verfahren zur Überwachung der Betriebsbereitschaft einer Sauerstoffmeßsonde wird die Sauerstoffmeßsonde mit einer Prüfspannung mit einem konstanten mittleren Wert der von der Sauerstoffmeßsonde erzeugbaren Spannung betastet. Die resultierende Spannung am Sondenausgang wird als Kontrollgröße der Betriebsbereitschaft zwei Vergleichsvorrichtungen zum Vergleich mit einem oberen und einem unteren Sondenwert jeweils entsprechend einer Mindestausgangsspannung der Sauerstoffmeßsonde zugeführt, und über ein Zeitglied wird eine Gemischsteuervorrichtung anstele der Gemischregeleinrichtung entsprechend dem Ausgangssignal der Vergleichsvorrichtung zu- oder abgeschaltet.

Bei einem anderen bekannten Verfahren (US-A-4 208 993) zur Überwachung der Betriebsbereitschaft einer Sauerstoffmeßsonde, bei welcher ebenfalls eine unter dem Einfluß des Sondenverhaltens resultierende Spannung von zwei Vergleichsschaltungen mit Schwellwertspannungen abgetastet und anschließend ausgewertet wird, wird zur Erfassung des die Sondenbereitschaft kennzeichnenden Sonden-Innenwiderstandes der Sauerstoffmeßsonde eine konstante Bezugsspannung entgegengeschaltet, wobei die der sich hierdurch ergebenden Ausgangsspannung entgegengeschalteten Schwellwertspannungen der Vergleichsschaltungen um vorgegebene Differenzwerte oberhalb und unterhalb der Bezugsspannung liegen, dabei werden die von den Vergleichsschaltungen abgegebenen, insgesamt drei logischen Schaltzuständen entsprechende Ausgangssignale von einer nachgeschalteten Auswerteschaltung zur Umschaltung zwischen Regelung auf Steuerung bzw. umgekehrt verarbeitet.

Aus dem Dokument EP-A-0 099 545 ist ferner eine Schaltungsanordnung bekannt, bei der zur Erkennung der Betriebsbereitschaft der Sauerstoffmeßsonde eine Vorspannung an die Sauerstoffmeßsonde angelegt und in aufeinanderfolgenden gleichförmigen Zeitabschnitten die Ausgangsspannung der Sauerstoffmeßsonde abgetastet wird. Durch Differenzbildung der abgetasteten Meßwerte ergibt sich die Änderungsgeschwindigkeit der Ausgangsspannung, wobei oberhalb eines festgelegten Wertes Betriebsbereitschaft angenommen wird.

Schließlich ist aus den Dokument US-A-4 132 193 die Verwendung von Integratoren in Abgastemperatur-Meßsystemen für Brennkraftmaschinen mit externer Gemischbildung bekannt.

Aufgabe der vorliegenden Erfindung ist es, ein Verfahren zur Erkennung der Betriebsbereitschaft einer Sauerstoffmeßsonde anzugeben, welches in zuverlässiger Weise mit einfachen und preiswerten Schaltungsanordnungen durchgeführt werden kann. Außerdem soll durch die Erfindung ermöglicht werden, die Erkennung mit einfachen Mitteln an die jeweils vorliegenden Verhältnisse anzupassen.

Das erfindungsgemäße Verfahren ist dadurch gekennzeichnet,
daß die Ausgangsspannung der Sauerstoffmeßsonde integriert und oberhalb eines vorgegebenen Wertes des Integrals Betriebsbereitschaft festgestellt wird, daß die Integrationszeitkonstante für die Integration vom. Innenwiderstand der Sauerstoffmeßsonde abhängt und daß nach dem Start der Brennkraftmaschine dem für die Integration der Ausgangsspannung der Sauerstoffmeßsonde vorgesehenen Integrator ein Spannungsanfangswert in mehreren kurzen Impulsen zugeführt wird.

Durch das erfindungsgemäße Verfahren ist eine einfache und zuverlässige Erkennung der Betriebsbereitschaft der Sauerstoffmeßsonde möglich. Es kann ebenfalls zur Überwachung der Sauerstoffmeßsonde während des Betriebes angewendet werden.

Eine Weiterbildung des erfindungsgemäßen Verfahrens besteht darin, daß ferner Betriebsbereitschaft nur festgestellt wird, solange ein zweiter vorgegebener Wert der Änderungsgeschwindigkeit

nicht überschritten wird. Diese Weiterbildung ermöglicht es, einen Kurzschluß der Sauerstoffmeßsonde zu erkennen.

Gemäß einer anderen Weiterbildung des erfindungsgemäßen Verfahrens ist vorgesehen, daß bei dem für die Integration der Ausgangsspannung der Sauerstoffmeßsonde vorgesehenen Integrator ein Eingang kurzzeitig weitgehend kurzgeschlossen wird.

Dadurch ist sichergestellt, daß auch während der Warmlaufphase ein Signal in Abhängigkeit vom Innenwiderstand der Sauerstoffmeßsonde integriert wird, so daß damit der Innenwiderstand meßbar ist.

Bei einer anderen Weiterbildung der Erfindung liegt der Anfangswert in einem mittleren Teil des Arbeitsbereichs der Ausgangsspannung des Integrators. Damit ist eine sichere Erkennung der Betriebsbereitschaft unabhängig davon möglich, ob in der Warmlaufphase fettes oder mageres Gemisch vorliegt und welche Spannung die Sauerstoffmeßsonde damit abgibt.

Gemäß einer anderen Weiterbildung der Erfindung kann die Änderungsgeschwindigkeit dadurch in einfacher Weise bestimmt werden, daß zur Auswertung der Ausgangsspannung des Integrators zwei eine Hysterese bildende Spannungsschwellen vorgesehen sind und daß die Zeit zwischen dem Zuführen des Anfangswertes und dem Überschreiten einer der Spannungsschwellen durch die Ausgangsspannung als Maß für die Änderungsgeschwindigkeit dient.

Eine andere Weiterbildung des Verfahrens besteht darin, daß während des Betriebes der Brennkraftmaschine wiederholt die Zuführung des Anfangswertes und die Messung der Änderungsgeschwindigkeit erfolgen Dadurch ist nicht nur eine Überwachung der Sauerstoffmeßsonde während der Warmlaufphase, sondern auch während des Betriebes möglich.

Eine Ausbildung des erfindungsgemäßen Verfahrens besteht darin, daß nach dem Start der Brennkraftmaschine wiederholt Messungen erfolgen, bis die Änderungsgeschwindigkeit auf oder über den vorgegebenen Wert gestiegen ist. Dabei kann die Zuführung des Anfangswertes und die Messung der Änderungsgeschwindigkeit nach vorgegebenen Zeitabständen wiederholt werden. Dabei können die Zeitabstände fest vorgegeben oder variabel sein.

Es kann jedoch auch vorgesehen sein, daß jeweils durch einen Sprung des Ausgangssignals der Sauerstoffmeßsonde die Zuführung des Anfangswertes und die Messung der Änderungsgeschwindigkeit ausgelöst werden. Um auch Messungen zu ermöglichen, wenn während der Warmlaufphase für längere Zeit ein Wechsel zwischen fettem und magerem Gemisch stattfindet, kön nen die Zuführung des Anfangswertes und die Messung der Änderungsgeschwindigkeit ferner ausgelöst werden, wenn nach einer vorgegebenen Zeit kein Sprung der Ausgangsspannung erfolgt.

Der vorgegebene Wert bzw. die vorgegebenen Werte können insbesondere dann leicht den Verhältnissen im Einzelfall angepaßt werden, wenn sie gemäß einer anderen Weiterbildung der Erfindung einem Speicher entnommen werden, dessen Inhalt veränderbar ist.

Eine vorteilhafte Schaltungsanordnung zur Durchführung des erfindungsgemäßen Verfahrens ist dadurch gekennzeichnet, daß an den Ausgang der Sauerstoffmeßsonde der Eingang eines Integrators angeschlossen ist, dessen Ausgang über einen Analog/Digital-Wandler mit dem Eingang eines Mikrocomputers verbunden ist.

Bei entsprechender Programmierung kann ein ohnehin für Regelzwecke eingesetzter Mikrocomputer zur Durchführung des erfindungsgemäßen Verfahrens durch Hinzufügung von nur wenigen elektronischen Bauelementen verwendet werden.

Eine vorteilhafte Ausgestaltung der erfindungsgemäßen Schaltungsanordnung besteht darin, daß der Eingang des Integrators ferner uber einen steuerbaren Schalter mit konstantem Potential verbindbar ist und daß dem steuerbaren Schalter ein Signal von einem Ausgang des Mikrocomputers zugeführt ist. Vorzugsweise ist der steuerbare Schalter ein Transistor.

Eine andere vorteilhafte Schaltungsanordnung zur Durchführung des erfindungsgemäßen Verfahrens ist dadurch gekennzeichnet, daß an den Ausgang der Sauerstoffmeßsonde der Eingang eines Integrators angeschlossen ist, dessen Ausgang über einen Analog/Digital-Wandler mit dem Eingang eines Mikrocomputers verbunden ist, daß der Eingang des Integrators ferner über einen steuerbaren Schalter mit konstantem Potential verbindbar ist, daß dem steuerbaren Schalter ein Signal von einem Ausgang des Mikrocomputers zugeführt ist und daß das konstante Potential dem Anfangswert entspricht.

Eine andere vorteilhafte Ausgestaltung der Erfindung besteht darin, daß der Integrator von einem Operationsverstärker gebildet ist, dessen invertierender Eingang den Eingang des Integrators bildet und über einen Kondensator mit dem Ausgang des Operationsverstärkers verbunden ist, und daß der nichtinvertierende Eingang mit einer konstanten Spannung beaufschlagt ist. Damit wird der Arbeitspunkt des Integrators festgelegt.

Es kann dabei vorgesehen sein, daß zwischen dem invertierenden Eingang des Operationsverstärkers und dem steuerbaren Schalter sowie zwischen dem invertierenden Eingang des Operationsverstärkers und dem Ausgang der Sauerstoffmeßsonde je ein abgleichbarer Widerstand angeordnet ist. Ferner kann der Kondensator mit dem Ausgang des

Operationsverstärkers über einen Spannungteiler verbunden sein.

Schließlich ist gemäß einer anderen Weiterbildung vorgesehen, daß der Kondensator derart dimensioniert ist, daß die Integrationszeitkonstante bei betriebsbereiter Sauerstoffmeßsonde im wesentlichen keinen Einfluß auf die Regelcharakteristik ausübt.

Dadurch ist es möglich, die erfindungsgemäße Schaltungsanordnung selbst zur Weiterleitung des Sondensignals zum Mikrocomputer zu verwenden. Es ist jedoch auch möglich, die Erkennung der Betriebsbereitschaft und eine Signalaufbereitung in getrennten Schaltungen durchzuführen.

Die Erfindung läßt zahlreiche Ausführungsformen zu. Eine davon ist schematisch in der Zeichnung an Hand mehrerer Figuren dargestellt und nachfolgend beschrieben. Es zeigt:

Fig. 1    ein Schaltbild einer ersten Schaltungsanordnung zur Durchführung des erfindungsgemäßen Verfahrens,

Fig. 2    Spannungs-Zeitdiagramme einiger bei der Schaltungsanordnung nach Fig. 1 auftretenden Spannungen und Signale,

Fig. 3    ein Schaltbild einer zweiten Schaltungsanordnung zur Durchführung des erfindungsgemäßen Verfahrens und

Fig. 4    Spannungs-Zeitdiagramme einiger bei der Schaltungsanordnung nach Fig. 3 auftretenden Spannungen und Signale.

Gleiche Teile sind in den Figuren mit gleichen Bezugszeichen versehen.

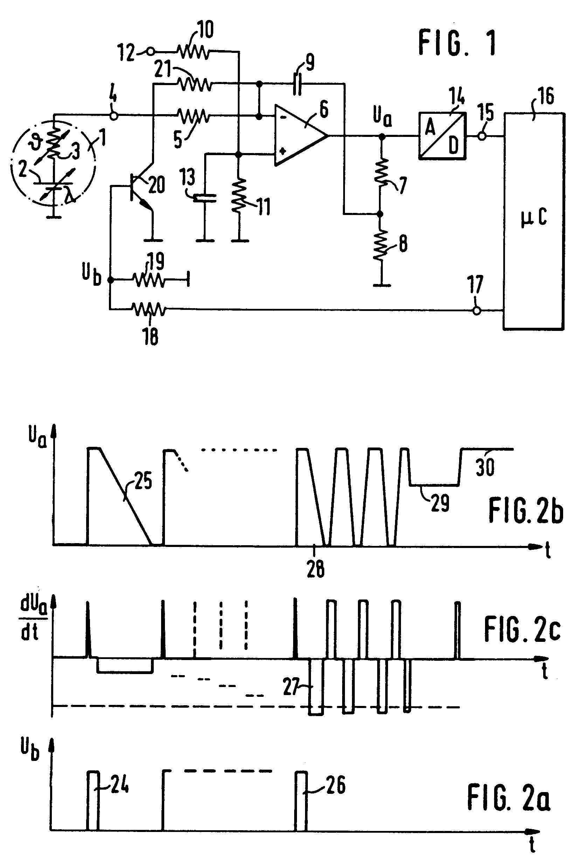

Die an sich bekannte Sauerstoffmeßsonde 1 ist in Fig. 1 lediglich als eine Spannungsquelle 2, deren Spannung von dem Sauerstoffanteil abhängt und deren Innenwiderstand 3 temperaturabhängig ist, dargestellt. Die Ausgangsspannung der Sauerstoffmeßsonde 1 wird über einen Anschluß 4 und einen abgleichbaren Widerstand 5 dem invertierenden Eingang eines Operationsverstärkers 6 zugeführt.

Der Operationsverstärker 6 bildet zusammen mit einem Spannungteiler 7, 8 und einem Kondensator 9 einen an sich bekannten Integrator. Dem nichtinvertierenden Eingang des Operationsverstärkers 6 wird über einen weiteren Spannungteiler 10, 11 die Hälfte der bei 12 zugeführten Betriebsspannung zugeführt. Zur Unterdrückung von Spannungsspitzen ist der nichtinvertierende Eingang ferner über einen Kondensator 13 mit Massepotential verbunden. Der Widerstand 11 des weiteren Spannungteilers 10, 11 ist abgleichbar ausgebildet, so daß die am nichtinvertierenden Eingang anliegende Spannung eingestellt werden kann.

Der Ausgang des Operationsverstärkers 6, der gleichzeitig den Ausgang des Integrators bildet, ist über einen Analog/Digital-Wandler 14 mit einem Eingang 15 eines Mikrocomputers 16 verbunden.

Ein Ausgang 17 des Mikrocomputers 16 ist über einen Spannungteiler 18, 19 mit der Basis eines Transistors 20 verbunden. Der Emitter des Transistors 20 ist an Massepotential und der Kolektor über einen abgleichbaren Widerstand 21 an den invertierenden Eingang des Operationsverstärkers 6 angeschlossen.

In an sich bekannter Weise ist der Mikrocomputer 16 mit Hilfe eines gespeicherten Programms sowie entsprechender im einzelnen nicht dargestellter Stellglieder dazu ausgelegt, die der Brennkraftmaschine zugeführte Kraftstoffmenge im Sinne einer vollständigen Verbrennung zu regeln. Dazu wird beispielsweise die Einspritzmenge durch Steuerung des Systemdrucks oder durch Steuerung der Einspritzdauer entsprechend geregelt. Derartige Systeme sind bekannt und brauchen im Zusammenhang mit der vorliegenden Erfindung nicht näher erläutert zu werden.

Bei dem dargestellten Ausführungsbeispiel ist der Mikrocomputer 16 durch ein weiteres Programm zum Betrieb der in Fig. 1 dargestellten Schaltung ausgelegt. Dazu wird, wie an Hand von Fig. 2 näher erläutert wird, nach einem Start der Brennkraftmaschine, wenn also die Lambda-Sonde kalt ist und einen hohen Innenwiderstand besitzt, der Transistor 20 (Fig. 1) kurzzeitig in den leitenden Zustand gesteuert. Dadurch nimmt der invertierende Eingang des Operationsverstärkers 6 Massepotential an, so daß die Ausgangsspannung die obere Grenze des Aussteuerbereichs einnimmt. Der Widerstand 21 hat einen derart geringen Wert, daß die dazu erforderliche Umladung des Kondensators 9 in kurzer Zeit möglich ist.

Nach der Rückflanke des Impulses 24 ist der Transistor 20 wieder nichtleitend, so daß sich die in Fig. 2b) dargestellte Ausgangsspannung des Operationsverstärkers 6 unter entsprechender Umladung des Kondensators 9 auf den durch die Ausgangsspannung der Sauerstoffmeßsonde vorgegebenen Wert einstellt. Da in den meisten Betriebsfällen die Brennkraftmaschine nach dem Starten ein zu fettes Gemisch erhält, also Sauerstoffmangel besteht, sinkt die Ausgangsspannung Ua von ihrem Maximalwert auf 0 entsprechend der in Fig. 2b) dargestellten Funktion 25. Da im kalten Zustand der Innenwiderstand 3 der Sauerstoffmeßsonde einen großen Wert aufweist, erfolgt der Abfall der Ausgangsspannung Ua sehr langsam. Bei einer praktisch ausgeführten Schaltungsanordnung wurden beispielsweise hierfür Zeiten in der Größenordnung von 20 ms benötigt.

Durch ein entsprechendes Programm im Mikrocomputer 16 (Fig. 1) wird die Änderungsgeschwindigkeit der Ausgangsspannung Ua ermittelt. Zur weiteren Verdeutlichung ist deshalb in Fig. 2c) die erste Ableitung nach der Zeit des in Fig. 2b) dargestellten Signals aufgetragen. Die waagrechte gestrichelte Linie stellt die Änderungsgeschwindigkeit dar, bei welcher die Betriebstemperatur als erreicht gilt. Da dieses nach der Messung nach dem ersten Impuls 24 nicht der Fall ist, werden vom Mikrocomputer 16 weitere Impulse abgegeben, die im einzelnen nicht dargestellt sind. Durch die mittlerweile eingetretene Erwärmung der Sauerstoffmeßsonde ist die Änderungsgeschwindigkeit der Ausgangsspannung Ua nach dem Impuls 26 größer als der vorgegebene Wert (siehe Kurventeil 27, Fig. 2c)).

Sobald die Änderungsgeschwindigkeit den vorgegebenen Wert überschreitet, wird im Mikrocomputer von einer Steuerung, die nach gespeicherten Kennlinienfeldern erfolgt, auf eine Regelung umgeschaltet. Von diesem Zeitpunkt 28 an ist die durch den Innenwiderstand und den zusätzlichen Abgleichwiderstand 5 sowie den Kondensator 9 gegebene Zeitkonstante so klein, daß die Regelcharakteristik des durch den Mikrocomputer, die Sonde, die erfindungsgemäße Schaltungsanordnung, die Brennkraftmaschine und die dazugehörigen Stellglieder gegebenen Regelkreises durch den Integrator grundsätzlich nicht beeinflußt wird. Abgesehen von weniger steilen Flanken und dem Vorzeichen entspricht die Ausgangsspannung Ua der verstärkten Ausgangsspannung der Sonde. Erfolgt eine Unterbrechung der Zuleitung von der Sonde zum Integrator, so erhöht sich die Zeitkonstante des Integrationsgliedes wesentlich, so daß für eine lange Zeit nach der Unterbrechung die zuvor bestehende Ausgangsspannung Ua bestehenbleibt (siehe Signalabschnitt 29). Im Falle eines Kurzschlusses der Sauerstoffmeßsonde steigt jedoch die Ausgangsspannung Ua auf ihren Maximalwert (siehe Signalabschnitt 30). Beides kann im Mikrocomputer ausgewertet werden.

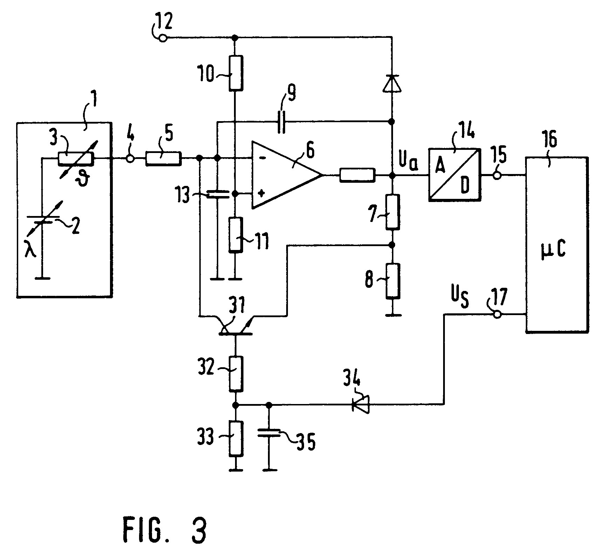

Zum Unterschied gegenüber der Schaltungsanordnung nach Fig. 1 wird bei der Schaltungsanordnung nach Fig. 3 der Integrator auf einen Anfangswert gesetzt, der im mittleren Teil des Aussteuerbereichs liegt. Dazu wird vom Ausgang 17 des Mikrocomputers 16 eine Steuerspannung Us der Basis eines Transistors 31 zugeführt, der den Ausgang des Spannungsteilers 37, 38 mit dem invertierenden Eingang des Operationsverstärkers 6 verbindet. Dabei dient die aus den Widerständen 32, 33, der Diode 34 und dem Kondensator 35 bestehende Schaltung zur Vorspannungserzeugung für den Transistor 31.

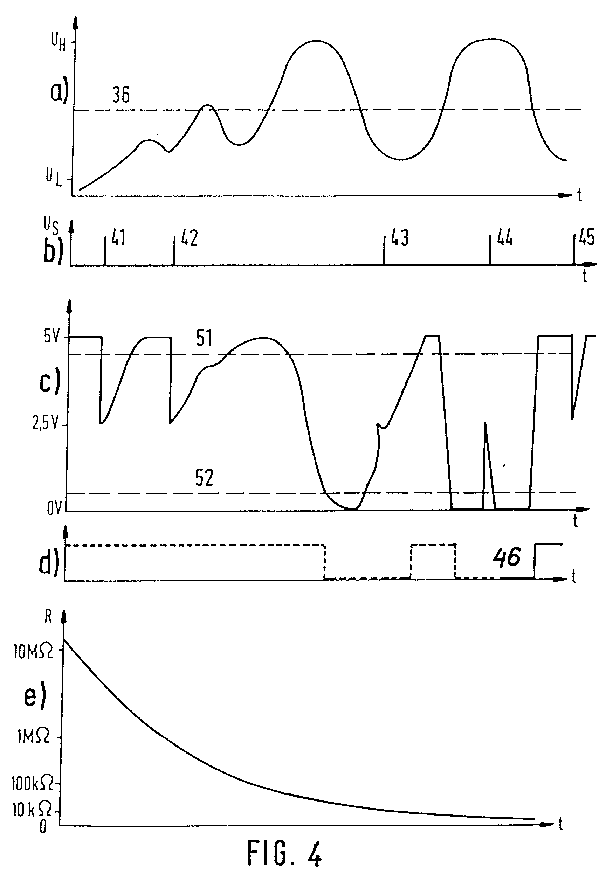

Die Funktion der Schaltungsanordnung nach Fig. 3 wird anhand von Fig. 4 näher erläutert. Dabei sind jeweils als Zeitdiagramm dargestellt:
In Fig. 4a) die Ausgangsspannung der Sauerstoffmeßsonde, in Fig. 4b) die vom Mikrocomputer abgegebene Steuerspannung Us, in Fig. 4c) die Ausgangsspannung des Integrators, in Fig. 4d) die ausgewertete Ausgangsspannung des Integrators und in Fig. 4e) der Innenwiderstand der Sauerstoffmeßsonde.

Die in Fig. 4a) dargestellte Ausgangsspannung der Sauerstoffmeßsonde nimmt bei Sauerstoffmangel, also bei zu fettem Gemisch, niedrige Werte, beispielsweise 0,1 V ein. Bei Sauerstoffüberschuß gibt die Sauerstoffmeßsonde eine Spannung ab, deren Maximalwert bei etwa 0,9 V liegt. Die Schwelle für den Integrator ist zwischen diese beiden Werte gelegt und in Fig. 4a) als gestrichelte Linie angedeutet.

In einem ersten Zeitraum nach dem Start der Brennkraftmaschine bei kalter Sauerstoffmeßsonde weist die Sauerstoffmeßsonde einen extrem hohen Innenwiderstand auf (siehe Fig. 4e)). Die Ausgangsspannung ist dann nicht auswertbar. Deshalb wird während dieses Zeitraums und im Falle einer Unterbrechung der Zuleitungen zur Sauerstoffmeßsonde ein Regler für die Gemischaufbereitung im Sinne einer Steuerung nach gespeicherten Kennlinienfeldern betrieben.

Nach einem Start der Brennkraftmaschine, wenn also die Sauerstoffmeßsonde kalt ist und einen hohen Innenwiderstand besitzt, wird der Transistor 31 (Fig. 3) durch einen Impuls 41 kurzzeitig in den leitenden Zustand gesteuert. Dadurch nimmt die Ausgangsspannung des Operationsverstärkers 6 einen Wert an, der in der Mitte des Arbeitsbereiches liegt.

Nach der Rückflanke des Impulses 41 ist der Transistor 31 wieder nichtleitend, so daß sich die in Fig. 4c) dargestellte Ausgangsspannung des Operationsverstärkers 6 auf 5 V steigt. Wegen des hohen Innenwiderstandes der Sauerstoffmeßsonde steigt die Ausgangsspannung des Operationsverstärkers 6 relativ langsam. Die Zeit zwischen dem Impuls 41 und dem Überschreiten der Spannungsschwelle 51 durch die Ausgangsspannung des Operationsverstärkers 6 wird gemessen. Liegt die gemessene Zeit über einem vorgegebenen Wert, so wird dadurch festgestellt, daß die Sauerstoffmeßsonde noch nicht betriebsbereit ist.

Beim nächsten Impuls 42 (Fig. 4b)) steigt die Ausgangsspannung des Operationsverstärkers 6 noch langsamer an, da hier einerseits der Innenwiderstand der Sauerstoffmeßsonde noch relativ groß ist und andererseits ihr Ausgangssignal sich in der Nähe der Schwelle 36 des Integrators befindet. Entsprechend wird auch nach diesem Impuls noch nicht auf einen Reglerbetrieb umgeschaltet. Danach tritt vorübergehend Sauerstoffüberschuß auf, so daß die Ausgangsspannung der Sauerstoffmeßsonde höhere Werte annimmt. Entsprechend sinkt das Ausgangssignal des Operationsverstärkers 6 und gelangt unter die Spannungsschwelle 52. In diesem Zusammenhang sei noch vermerkt, daß die Spannungsschwellen 51 und 52 und ein Vergleich der Ausgangsspannung des Operationsverstärkers 6 mit diesen Spannungsschwellen im Mikrocomputer durch ein entsprechendes Programm und mit gespeicherten Werten für die Spannungsschwellen 51, 52 realisiert werden.

Nach dem vom Mikrocomputer 16 abgegebenen Impuls 43 erfolgt ebenfalls noch kein schneller Spannungsanstieg, da der Impuls etwa in einen Vorzeichenwechsel der Sauerstoffmeßsonde fällt. Inzwischen ist jedoch die Sauerstoffmeßsonde so weit erwärmt, daß ihr Innenwiderstand klein geworden ist. Nach dem Impuls 44 fällt daher die Ausgangsspannung des Operationsverstärkers 6 schnell wieder auf null und unterschreitet die Schwelle 52 innerhalb des vorgegebenen Zeitraumes. Es wird daraus auf eine Betriebsbereitschaft der Sauerstoffmeßsonde geschlossen und von einer Steuerung auf eine Regelung umgeschaltet. Nach einem Sprung der Ausgangsspannung der Sauerstoffmeßsonde durch eine Gemisch-Anreicherung überschreitet die Ausgangsspannung des Operationsverstärkers 6 die obere Schwelle 51. Bei dem darauf folgenden Impuls 45 wird die Ausgangsspannung wieder auf den mittleren Wert von 2,5 V gesetzt und steigt dann schnell wieder auf den oberen Wert von 5 V.

Die Schwellen 51, 52 bewirken eine Hysterese derart, daß das letztlich fur die Regelung auszuwertende Signal einen ersten Wert H annimmt, wenn die Ausgangsspannung des Operationsverstärkers 6 die obere Schwelle 51 überschreitet, und den zweiten Wert L einnimmt, wenn die Schwelle 52 unterschritten wird. Fig. 4d) zeigt dieses Signal, wobei es bis zum Zeitpunkt 46 punktiert dargestellt ist, da es erst von diesem Zeitpunkt an für eine Regelung genutzt wird.

Da das Zuführen des Anfangswertes für den Integrator die Erzeugung des in Fig. 4b) dargestellten Signals und damit die Regelfunktion in keiner Weise beeinträchtigt, kann die Prüfung der Betriebsbereitschaft der Sauerstoffmeßsonde nicht nur in einer Warmlauf phase, sondern auch laufend während des Betriebes erfolgen, so daß auftretende Fehler, beispielsweise eine Unterbrechung der Zuleitung, sofort angezeigt werden können. Eine Umschaltung des Reglers auf einen Betrieb im Sinne einer Steuerung kann dann ebenfalls erfolgen.

Die Impulse 41 bis 45 können regelmäßig vom Mikrocomputer 16 abgegeben werden, es ist jedoch auch eine Auslösung durch Sprünge der Ausgangsspannung der Sauerstoffmeßsonde möglich.

**Patentansprüche**

1. Verfahren zur Erkennung der Betriebsbereitschaft einer Sauerstoffmeßsonde, die im Abgaskanal einer Brennkraftmaschine angeordnet ist und zusammen mit einer Regeleinrichtung zur Regelung der Gemischaufbereitung für die Brennkraftmaschine dient, wobei die Änderungsgeschwindigkeit der Ausgangsspannung der Sauerstoffmeßsonde in aufeinanderfolgenden Zeitabschnitten errechnet und oberhalb eines vorgegebenen Wertes der errechneten Änderungsgeschwindigkeit Betriebsbereitschaft festgestellt wird,
dadurch gekennzeichnet,
daß die Ausgangsspannung der Sauerstoffmeßsonde integriert und oberhalb eines vorgegebenen Wertes des Integrals Betriebsbereitschaft festgestellt wird, daß die Integrationszeitkonstante für die Integration vom Innenwiderstand der Sauerstoffmeßsonde abhängt und daß nach dem Start der Brennkraftmaschine dem für die Integration der Ausgangsspannung der Sauerstoffmeßsonde vorgesehenen Integrator ein Spannungsanfangswert in mehreren kurzen Impulsen zugeführt wird.

2. Verfahren nach Anspruch 1, dadurch gekennzeichnet, daß ferner Betriebsbereitschaft nur festgestellt wird, solange ein zweiter vorgegebener Wert der Änderungsgeschwindigkeit nicht überschritten wird.

3. Verfahren nach Anspruch 1, dadurch gekennzeichnet, daß ein Eingang des Integrators kurzzeitig weitgehend kurzgeschlossen wird.

4. Verfahren nach Anspruch 1, dadurch gekennzeichnet, daß der Spannungsanfangswert in einem mittleren Teil des Arbeitsbereichs der Ausgangsspannung des Integrators liegt.

5. Verfahren nach einem der Ansprüche 1 bis 4, dadurch gekennzeichnet, daß zur Auswertung der Ausgangsspannung des Integrators zwei eine Hysterese bildende Spannungsschwellen vorgesehen sind und daß die Zeit zwischen dem Zuführen des Anfangswertes und dem überschreiten einer der Spannungsschwellen durch die Ausgangsspannung als Maß für die Änderungsgeschwindigkeit dient.

6. Verfahren nach einem der Ansprüche 1 bis 5, dadurch gekennzeichnet, daß während des Betriebes der Brennkraftmaschine wiederholt die Zuführung des Anfangswertes und die Messung der Änderungsgeschwindigkeit erfolgen.

7. Verfahren nach einem der Ansprüche 1 bis 5, dadurch gekennzeichnet, daß nach dem Start der Brennkraftmaschine wiederholt die Zuführung des Anfangswertes und Messungen der Änderungsgeschwindigkeit erfolgen, bis die Änderungsgeschwindigkeit auf oder über den vorgegebenen Wert gestiegen ist.

8. Verfahren nach einem der Ansprüche 6 oder 7, dadurch gekennzeichnet, daß die Zuführung des Anfangswertes und die Messung der Änderungsgeschwindigkeit nach vorgegebenen Zeitabständen wiederholt werden.

9. Verfahren nach einem der Ansprüche 6 oder 7, dadurch gekennzeichnet, daß jeweils durch einen Sprung des Ausgangssignals der Sauerstoffmeßsonde die Zuführung des Anfangswertes und die Messung der Änderungsgeschwindigkeit ausgelöst werden.

10. Verfahren nach Anspruch 9, dadurch gekennzeichnet, daß die Zuführung des Anfangswertes und die Messung der Änderungsgeschwindigkeit ferner ausgelöst werden, wenn nach einer vorgegebenen Zeit kein Sprung der Ausgangsspannung erfolgt.

11. Verfahren nach einem der vorhergehenden Ansprüche, dadurch gekennzeichnet, daß der vorgegebene Wert bzw. die vorgegebenen Werte der Änderungsgeschwindigkeit einem Speicher entnommen werden, dessen Inhalt veränderbar ist.

12. Schaltungsanordnung zur Durchführung des Verfahrens nach einem der Ansprüche 1 bis 11, dadurch gekennzeichnet, daß an den Ausgang der Sauerstoffmeßsonde (1) der Eingang eines Integrators angeschlossen ist, dessen Ausgang über einen Analog/Digital-Wandler (14) mit dem Eingang (15) eines Mikrocomputers (16) verbunden ist, daß der Eingang des Integrators ferner über einen steuerbaren Schalter (20, 31) führt, dessen Steuereingang nach dem Start der Brennkraftmaschine in mehreren kurzen Impulsen ein Signal vom Ausgang (17) des Mikrocomputers (16) zugeführt ist.

13. Schaltungsanordnung nach Anspruch 12, dadurch gekennzeichnet, daß der Eingang des Integrators ferner über den steuerbaren Schalter (20) mit konstantem Potential verbindbar ist.

14. Schaltungsanordnung nach Anspruch 12, dadurch gekennzeichnet, daß der Eingang des Integrators ferner über den steuerbaren Schalter (31) mit dem Ausgang verbindbar ist.

15. Schaltungsanordnung nach einem der Ansprüche 13 oder 14, dadurch gekennzeichnet, daß der steuerbare Schalter (20, 31) ein Transistor ist.

16. Schaltungsanordnung nach einem der Ansprüche 12 bis 15, dadurch gekennzeichnet, daß der Integrator von einem Operationsverstärker (6) gebildet ist, dessen invertierender Eingang den Eingang des Integrators bildet und über einen Kondensator (9) mit dem Ausgang des Operationsverstärkers (6) verbunden ist, und daß der nichtinvertierende Eingang mit einer konstanten Spannung beaufschlagt ist.

17. Schaltungsanordnung nach Anspruch 13, dadurch gekennzeichnet, daß zwischen dem invertierenden Eingang des Operationsverstärkers (6) und dem steuerbaren Schalter (20) sowie zwischen dem invertierenden Eingang des Operationsverstärkers (6) und dem Ausgang der Sauerstoffmeßsonde (1) je ein abgleichbarer Widerstand (5, 21) angeordnet ist.

18. Schaltungsanordnung nach Anspruch 13, dadurch gekennzeichnet, daß der Kondensator (9) mit dem Ausgang des Operationsverstärkers (6) über einen Spannungsteiler (7, 8) verbunden ist.

19. Schaltungsanordnung nach einem der Ansprüche 12 bis 18, dadurch gekennzeichnet, daß der Kondensator derart dimensioniert ist, daß die Integrationszeitkonstante bei betriebsbereiter Sauerstoffmeßsonde im wesentlichen keinen Einfluß auf die Regelcharakteristik ausübt.

**Claims**

1. Method for detecting the readiness for operation of an oxygen sensor which is arranged in the exhaust-gas duct of an internal combustion engine and, together with a control device, serves for the closed-loop control of mixture preparation for the internal combustion engine, the rate of change of the output voltage of the oxygen sensor in successive time periods being calculated and readiness for operation being established above a predetermined value of the calculated rate of change, characterised in that the output voltage of the oxygen sensor is integrated and readiness for operation is established above a predetermined value of the integral, in that the integration time constant for the integration depends on the internal resistance of the oxygen sensor and in that, following the start of the internal combustion engine, an initial voltage value is supplied in a plurality of short pulses to the integrator provided for integrating the output voltage of the oxygen sensor.

2. Method according to Claim 1, characterised in that readiness for operation is furthermore only established for as long a second predetermined value of the rate of change is not exceeded.

3. Method according to Claim 1, characterised in that one input of the integrator is largely short-circuited for a brief period.

4. Method according to Claim 1, characterised in that the initial voltage value lies in a central part of the working range of the output voltage of the integrator.

5. Method according to one of Claims 1 to 4, characterised in that two voltage thresholds forming a hysteresis are provided for evaluating the output voltage of the integrator and in that the time between the supply of the initial value and the exceeding of one of the voltage thresholds by the output voltage serves as a measure of the rate of change.

6. Method according to one of Claims 1 to 5, characterised in that the supply of the initial value and the measurement of the rate of change are performed repeatedly during the operation of the internal combustion engine.

7. Method according to one of Claims 1 to 5, characterised in that, following the start of the internal combustion engine, the supply of the initial value and measurements of the rate of change are performed repeatedly until the rate of change has risen to or above the predetermined value.

8. Method according to one of Claims 6 or 7, characterised in that the supply of the initial value and the measurement of the rate of change are repeated after predetermined time intervals.

9. Method according to one of Claims 6 or 7, characterised in that the supply of the initial value and the measurement of the rate of change are each triggered by a jump in the output signal of the oxygen sensor.

10. Method according to Claim 9, characterised in that the supply of the initial value and the measurement of the rate of change are furthermore triggered if no jump in the output voltage occurs after a predetermined time.

11. Method according to one of the preceding claims, characterised in that the predetermined value or the predetermined values of the rate of change are taken from a memory, the contents of which can be changed.

12. Circuit arrangement for carrying out the method according to one of Claims 1 to 11, characterised in that connected to the output of the oxygen sensor (1) is the input of an integrator, the output of which is connected via an analog/digital convertor (14) to the input (15) of a microcomputer (16), in that the input of the integrator furthermore leads via a controllable switch (20, 31), the control input of which is supplied in a plurality of short pulses with a signal by the output (17) of the microcomputer (16) following the start of the internal combustion engine.

13. Circuit arrangement according to Claim 12, characterised in that the input of the integrator can furthermore be connected to constant potential via the controllable switch (20).

14. Circuit arrangement according to Claim 12, characterised in that the input of the integrator can furthermore be connected to the output via the controllable switch (31).

15. Circuit arrangement according to one of Claims 13 or 14, characterised in that the controllable switch (20, 31) is a transistor.

16. Circuit arrangement according to one of Claims 12 to 15, characterised in that the integrator is formed by an operational amplifier (6), the inverting input of which forms the input of the integrator and is connected via a capacitor (9) to the output of the operational amplifier (6), and in that the non-inverting input is subjected to a constant voltage.

17. Circuit arrangement according to Claim 13, characterised in that one trimmable resistor (5, 21) in each case is arranged between the inverting input of the operational amplifier (6) and the controllable switch (20) and between the inverting input of the operational amplifier (6) and the output of the oxygen sensor (1).

18. Circuit arrangement according to Claim 13, characterised in that the capacitor (9) is connected to the output of the operational amplifier (6) via a voltage divider (7, 8).

19. Circuit arrangement according to one of Claims 12 to 18, characterised in that the capacitor is dimensioned in such a way that the integration time constant when the oxygen sensor is ready for operation exerts essentially no influence on the control characteristic.

## Revendications

1. Procédé de détection de l'état de préparation au fonctionnement d'une sonde de mesure d'oxygène qui est disposée dans la tuyauterie d'échappement d'un moteur à combustion interne et qui sert, en même temps qu'un système de réglage, au réglage du mélange préparé pour le moteur à combustion interne, la vitesse de variation de la tension de sortie de la sonde de mesure d'oxygène étant calculée à des intervalles de temps successifs et l'état de préparation au fonctionnement étant constaté lorsqu'une valeur, fixée à l'avance, de la vitesse de variation calculée se trouve dépassée, procédé caractérisé en ce que la tension de sortie de la sonde de mesure d'oxygène est soumise à intégration et en ce qu'au-dessus d'une valeur, fixée à l'avance, de l'intégrale, l'état de préparation au fonctionnement est constaté ; en ce que la constante de temps de l'intégration dépend de la résistance interne de la sonde de mesure d'oxygène et en ce qu'après le démarrage du moteur à combustion interne, une valeur initiale de tension est appliquée sous forme de plusieurs impulsions courtes, au dispositif d'intégration prévu pour l'intégration de la tension de sortie de la sonde de mesure d'oxygène.

2. Procédé selon la revendication 1, caractérisé en ce qu'en outre on ne constate que l'état de préparation au fonctionnement est atteint que tant qu'une deuxième valeur fixée à l'avance pour la vitesse de variation n'est pas dépassée.

3. Procédé selon la revendication 1, caractérisé en ce qu'une entrée du dispositif d'intégration est brièvement mise franchement en court-circuit.

4. Procédé selon la revendication 1, caractérisé en ce que la valeur de tension initiale se situe dans la partie médiane du domaine de travail de la tension de sortie du dispositif d'intégration.

5. Procédé selon une des revendications 1 à 4, caractérisé en ce qu en vue de l'exploitation de la tension de sortie du dispositif d'intégration, on prévoit deux seuils de tension formant une hystérésis ; et en ce que le temps qui s'écoule entre l'application de la valeur initiale et le dépassement de l'un des seuils de tension par la tension de sortie sert de mesure de la vitesse de variation.

6. Procédé selon une des revendications 1 à 5, caractérisé en ce que, pendant le fonctionnement du moteur à combustion interne, l'application de la valeur initiale et la mesure de la vitesse de variation sont effectuées de façon répétée.

7. Procédé selon une des revendications 1 à 5, caractérisé en ce qu'après le démarrage du moteur à combustion interne, l'application de la valeur initiale et la mesure de la vitesse de variation sont effectuées de façon répétée jusqu'à ce que la vitesse de variation ait atteint ou dépassé la valeur fixée à l'avance.

8. Procédé selon une des revendications 6 ou 7, caractérisé en ce que l'application de la valeur initiale et la mesure de la vitesse de variation sont répétées à des intervalles fixés à l'avance.

9. Procédé selon une des revendications 6 ou 7, caractérisé en ce que l'application de la valeur initiale et la mesure de la vitesse de variation sont déclenchées par une saute du signal de sortie de la sonde de mesure d'oxygène.

10. Procédé selon la revendication 9, caractérisé en ce que l'application de la valeur initiale et la mesure de la vitesse de variation sont en outre déclenchées lorsqu'après un temps fixé à l'avance, il ne s'est pas produit de saute de la tension de sortie.

11. Procédé selon une des revendications précédentes, caractérisé en ce que la valeur fixée à l'avance, ou les valeurs fixées à l'avance, de la vitesse de variation sont prélevées dans une mémoire dont le contenu peut être modifié.

12. Agencement de circuit pour l'exécution du procédé selon une des revendications 1 à 11, caractérisé en ce que la sortie de la sonde de mesure d'oxygène est connectée à l'entrée d'un dispositif d'intégration dont la sortie est connectée, par l'intermédiaire d'un convertisseur analogique/numérique (14), à l'entrée (15) d'un microordinateur (16); en ce que l'entrée du dispositif d'intégration est raccordée en outre,par l'intermédiaire d'un interrupteur (20, 31) susceptible d'être commandé, à l'entrée de commande duquel est appliqué, après le démarrage du moteur à combustion interne, en plusieurs impulsions brèves, un signal provenant de la sortie (17) du microordinateur (16).

13. Agencement de circuit selon la revendication 12, caractérisé en ce que l'entrée du dispositif d'intégration peut en outre être connectée, par l'intermédiaire de l'interrupteur (20) susceptible d'être commandé, à une source de potentiel constant.

14. Agencement de circuit selon la revendication 12, caractérisé en ce que l'entrée du dispositif d'intégration peut en outre être connectée à la sortie par l'intermédiaire de l'interrupteur (13)-susceptible d'être commandé.

15. Agencement de circuit selon une des revendications 13 ou 14, caractérisé en ce que l'interrupteur (20, 31) susceptible d'être commandé est un transistor.

16. Agencement de circuit selon une des revendications 12 à 15, caractérisé en ce que le dispositif d'intégration est constitué par un amplificateur opérationnel (6) dont l'entrée inversible forme l'entrée du dispositif d'intégration et est connectée par l'intermédiaire d'un condensateur (9) à la sortie de l'amplificateur opérationnel (6) et en ce que l'entrée non inversible est soumise à une tension constante.

17. Agencement de circuit selon la revendication 13, caractérisé en ce qu'entre l'entrée inversible de l'amplificateur opérationnel (6) et l'interrupteur (20) susceptible d'être commandé, ainsi qu'entre l'entrée inversible de l'amplificateur opérationnel (6) et la sortie de la sonde de mesure d'oxygène (1), on monte dans chaque cas une résistance (5, 21) réglable.

18. Agencement de circuit selon la revendication 13, caractérisé en ce que le condensateur (9) est connecté à la sortie de l'amplificateur opérationnel (6) par l'intermédiaire d'un diviseur (7, 8) de tension.

19. Agencement de circuit selon une des revendications 12 à 18, caractérisé en ce que le condensateur est dimensionné de façon telle que la constante de temps d'intégration n'exerce, lorsque la sonde de mesure d'oxygène est en un état où elle est prête à fonctionner, sensiblement aucune influence sur la caractéristique de réglage.

FIG. 1

FIG. 2b

FIG. 2c

FIG. 2a

FIG. 3

FIG. 4